# EUROPÄISCHE PATENTSCHRIFT

(11) **EP 0 538 673 B1**
(45) Veröffentlichungstag und Bekanntmachung des Hinweises auf die Patenterteilung: **07.01.1998**
(21) Anmeldenummer: 92117083.3
(22) Anmeldetag: 07.10.1992
(51) Int. Cl.: C08G 18/10, C08G 18/66, C08G 18/76, A61F 2/00, A61M 5/00

(54) **Transparente, heissdampfsterilisierbare, nicht zytotoxische, im wesentlichen kompakte Polyurethan-Vergussmassen, Verfahren zu ihrer Herstellung und ihre Verwendung, insbesondere für medizinisch-technische Artikel**
Clear, vapour-sterilisable, non-zytotoxic, essentially compact polyurethane casting compounds, process for their preparation and their use, especially for medical-technical articles
Masses à couler de polyuréthane substantiellement compacts, clairs et stérilisables à la vapeur chaude, non zytotoxique, procédé pour leur préparation et leur utilisation, en particulier pour des articles techniques médicinaux

(30) Priorität: 21.10.1991 DE 4134693
(43) Veröffentlichungstag der Anmeldung: 28.04.1993
(73) Patentinhaber: BASF Aktiengesellschaft, 67063 Ludwigshafen (DE)
(72) Erfinder: Horn, Peter, Dr., W-6900 Heidelberg (DE); Hinz, Werner, Dr., W-6710 Frankenthal (DE); Knoblauch, Georg, W-8000 München 60 (DE); Ramsteiner, Falko, Dr., W-6700 Ludwigshafen (DE); Knorr, Gottfried, Dr., O-7817 Schwarzheide (DE)

(56) Entgegenhaltungen:
- EP-A- 0 045 393
- EP-A- 0 393 545
- EP-A- 0 413 265
- DE-A- 2 749 491
- FR-A- 2 283 917
- US-A- 3 016 404
- US-A- 4 256 617

## Beschreibung

Erfindungsgegenstand sind transparente, heißdampfsterilisierbare, nicht zytotoxische, im wesentlichen kompakte Polyurethan- - im folgenden auch abgekürzt PU genannt - -Vergußmassen, die hergestellt werden durch Umsetzung von
a) modifizierten Diphenylmethan-diisocyanaten mit einer Viskosität bei 23°C von 100 bis 8000 mPas mit
b) Verbindungen mit mindestens zwei reaktiven Wasserstoffatomen
   in Abwesenheit oder vorzugsweise Gegenwart von
c) Katalysatoren
   wobei man als Aufbaukomponente (b) eine Mischung verwendet, die enthält, bezogen auf das Gesamtgewicht von (b1) und (b2),
   b1) 1 bis 20 Gew.-% mindestens eines Polyether-polyols mit einer mittleren Funktionalität von 3 bis 8, einer Hydroxylzahl von 200 bis 1000 und einem Gehalt an Alkaliionen von 150 bis 1200 ppm und
   b2) 99 bis 80 Gew.-% Ricinusöl und/oder mindestens eines Polyether-polyols mit einer Funktionalität von 2 bis 3, einer Hydroxylzahl von 90 bis 200 und einem Alkaliionengehalt von kleiner als 10 ppm.

PU-Gießsysteme sind bekannt und werden z. B. zusammenfassend beschrieben im Kunststoff-Handbuch "Polyurethane", Band 7, 2. Auflage, 1983, Seiten 392ff, herausgegeben von Dr. G. Oertel, im Carl Hanser Verlag, München, Wien.

Die Verwendung von PU-Vergußmassen zur Herstellung von Formteilen für medizinisch-technische Geräte, insbesondere als Einbettwerkstoff für Hohlfasern in Dialysatoren, ist ebenfalls nicht neu und wird aufgrund der einfachen Handhabung von Pu-Vergußmassen sowie deren geringer Schrumpfung während des Aushärtungsprozesses als vorteilhaft empfohlen. Bekannt sind z.B. folgende PU-Formulierungen speziell zur Einbettung von Hohlfasern.

In der US-A-3 962 094 werden katalysatorfreie Vergußmassen aus Rizinusöl-4,4'-MDI-, -Toluylen-diisocyanat- oder -Phenylen-diisocyanat-Prepolymeren mit endständigen NCO-Gruppen und einem Vernetzungsmittel, das enthält Rizinusöl und/oder einen Ester aus einem mindestens vierwertigen Alkohol und einer Hydroxy- oder Epoxygruppen aufweisenden aliphatischen Carbonsäure mit mindestens 12 Kohlenstoffatomen, beschrieben.

Nach Angaben der DE-A-2 749 491 (US-A-4 170 559) bestehen die katalysatorfreien Vergußmassen aus einem Prepolymeren, hergestellt aus Rizinusöl und Polyoxypropylen-glykol sowie 4,4'-MDI, und einem Vernetzungsmittel auf der Grundlage eines Esters aus einem mehrwertigen Alkohol mit 2 oder 3 Hydroxylgruppen und einer aliphatischen Carbonsäure mit mindestens 12 C-Atomen und einer oder mehreren Hydroxyl- und/oder Epoxygruppen. Als geeignete Polyisocyanate zur Herstellung der Prepolymeren werden ferner genannt: 2,4- und 2,6-Toluylen-diisocyanat oder Phenylen-diisocyanat. Als Vernetzungsmittel kommen außerdem Monoester und/oder Diester von Ethylenglykol und Ricinolsäure, Trimethylolpropan oder -ethan in Betracht.

Physiologisch unbedenkliche PU-Formstoffe, insbesondere zum Einbetten von Hohlfasern in Dialysatoren, werden nach Angaben der DD-A-251 565 hergestellt durch Umsetzung hochreaktiver, niedrigviskoser und lagerstabiler Mischprepolymerer, bestehend aus festen, hochreaktiven aromatischen Diisocyanaten und weniger reaktiven, flüssigen Diisocyanaten im Gewichtsverhältnis von 1:5 bis 5:1 und Polyolen, mit Polyolen aus der Gruppe Rizinusöl und/oder dessen Umesterungsprodukte, hochreiner Polyester und Polyoxytetramethylen-glykol. PU-Vergußmassen aus einem Isocyanatendgruppen aufweisenden PU-Prepolymeren und einer N,N,N',N'-Tetrakis(2-hydroxypropyl)ethylendiamin enthaltenden Polyolmischung sind Gegenstand des US-Patents Nr. 4 224 164. Nach Angaben der US-A-4 742 112 finden zur Herstellung von PU-Vergußmassen für elektrische Geräte als Polyolkomponente Mischungen aus 10 bis 60 Gew.% eines Ricinolsäureesters und 90 bis 40 Gew.% eines C₂- bis C₆-Kohlenwasserstoffpolymeren mit mindestens einer Hydroxylgruppe Verwendung. Zweikomponenten PU-Formulierungen, die im gehärteten Zustand nicht zytotoxisch sind und sich als Vergußmassen für Trennvorrichtungen eignen, bestehen nach Angaben der DE-A-3 048 529 (US-A-4 332 927) aus mindestens einem NCO-terminiertem Prepolymeren, mindestens einem Polyol und einer katalytisch wirksamen Mengen einer dicarboxylierten Dialkylzinnverbindung. Mit Zinn-Schwefel-Verbindungen katalysierte PU-Vergußmassen zur Einbettung von Cellulosehohlfasern in Dialysatoren werden beschrieben in der DD-A-155 777.

Die vorgenannten PU-Vergußmassen können zu medizinisch-technischen Geräten oder Formteilen hierfür verarbeitet und vor ihrem Gebrauch mit Ethylenoxid oder/und mit γ-Strahlen sterilisiert werden. Nachteilig an dieser Art der Sterilisation ist jedoch, daß verbleibende Ethylenoxidspuren bei Patienten teilweise Allergien auslösen können und die γ-Strahlen nicht identifizierbare Spaltprodukte bilden können, so daß beim Patienten ein gewisses Gesundheitsrisiko, verursacht durch die Dialyse, nicht vollständig ausgeschlossen werden kann. Die nach dem Stand der Technik bekannten Vergußmassen sind ferner nicht ausreichend temperatur- und chemikalienresistent, so daß diese keiner Heißdampfsterilisation bei einer Temperatur von 121°C über einen Zeitraum von 20 Minuten unterworfen werden können.

Ein anderer gravierender Nachteil besteht darin, daß die bekannten PU-Vergußmassen nicht mit jedem Fasertyp verarbeitbar sind. So werden beispielsweise Cellulosefasern von PU-Vergußmassen auf Rizinusölbasis angegriffen und geschädigt. Außerdem treten bei der Verarbeitung der zum Stande der Technik gehörenden PU-Vergußmassensysteme vielfach Schwierigkeiten während des Produktionsablaufs auf. Die hergestellten Vergußmassen lassen sich zwar unmittelbar nach dem Verguß und innerhalb eines Zeitraums von ungefähr 30 Minuten danach schneiden; sie härten jedoch sehr rasch nach, so daß die Formkörper, vorzugsweise Dialysefilter bereits nach 24 Stunden nicht mehr geschnitten werden können. Dieses nachteilige Verhalten führt insbesondere bei Produktionsstörungen und an Arbeitsenden zu Produkteinbußen. Finden zur Herstellung der PU-Vergußmassen PU-Formulierungen Verwendung, die als Aufbaukomponenten primäre, sekundäre und/oder tertiäre Aminogruppen und reaktive Wasserstoffatome gebunden enthalten, so sind die erhaltenen Vergußmassen instabil gegen eine Sterilisation mit Peressigsäure.

Zur Überwindung dieser Nachteile werden in der EP-A-0 393 545 und EP-A-0 413 265 transparente, heißdampfsterilisierbare, im wesentlichen kompakte PU-Vergußmassen beschrieben, die unter Verwendung von mit ausgewählten Verbindungen modifizierten Diphenylmethandiisocyanaten (MDI) hergestellt werden. nach Angaben der EP-A-0 393 545 werden geeignete, bei 23°C flüssige, modifizierte MDI hergestellt durch Umsetzung einer MDI-Isomerenmischung, die, bezogen auf 100 Gew.-Teile, besteht aus 60 bis 90 Gew.-Teilen 4,4'-MDI, 40 bis 8 Gew.-Teilen 2,4'-MDI und 0 bis 5 Gew.-Teilen 2,2'-MDI, mit einem mit Glycerin und/oder Trimethylpolypropan gestarteten Polyoxypropylen-polyol mit einem Molekulargewicht von 350 bis 800. Die EP-A-0 413 265 beschreibt die Verwendung von modifizierten MDI's, die erhalten werden durch Reaktion von 4,4'-MDI oder 2,4'-MDI oder MDI-Isomerenmischungen mit ausgewählten speziellen, mindestens tetrafunktionellen Polyoxypropylenpolyolen und/oder Polyoxypropylen-polyoxyethylen-polyolen mit einem Gehalt an polymerisierten Ethylenoxideinheiten von bis 80 Gew.-% zur Herstellung der PU-Vergußmassen. Aus den vorgenannten Aufbaukomponenten hergestellt PU-Vergußmassen besitzen eine erhöhte Temperaturbeständigkeit und verbesserte Hydrolysebeständigkeit. Vorteilhaft ist ferner, daß die maximale Härtungstemperatur, die durch die freiwerdende Reaktionswärme der Polyadditionsreaktion erreicht wird, deutlich gesenkt werden kann. Es hat sich jedoch auch gezeigt, daß nach mehrmaligen Sterilisationen Haftungsprobleme zwischen PU-Vergußmassen und anderen Werkstoffen, wie z. B. Polycarbonaten, auftreten können. Beispielsweise können sich die PU-Vergußmassen vom Gehäuseteil eines Dialysators ablösen. Solche Ablösungserscheinungen traten insbesondere dann auf, wenn durch Störungen bei der Heißdampfsterilisation die Temperatur von 121°C und/oder die Sterilisationszeit von 20 Minuten überschritten wurde.

Die Aufgabe der vorliegenden Erfindung bestand darin, die bekannten PU-Vergußmassen, vorzugsweise die in den EP-A-0 393 545 und EP-A-0 413 265 beschriebenen, durch geeignete Maßnahmen zu verbessern. Verbessert werden sollte insbesondere die Haftung zwischen den Pu-Vergußmassen und anderen Werkstoffen, so daß zumindest kurzzeitige Störungen bei der Sterilisation keine Ablösung der PU-Vergußmassen bewirken.

Diese Aufgabe könnte überraschenderweise gelöst werden durch transparente, heißdampfsterilisierbare, nicht zytotoxische, im wesentlichen kompakte Polyurethan- - im folgenden auch abgekürzt PU genannt - -Vergußmassen, die hergestellt werden durch Umsetzung von
a) modifizierten Diphenylmethan-diisocyanaten mit einer Viskosität bei 23°C von 100 bis 8000 mPas mit
b) Verbindungen mit mindestens zwei reaktiven Wasserstoffatomen
   in Abwesenheit oder vorzugsweise Gegenwart von
c) Katalysatoren
   wobei man als Aufbaukomponente (b) eine Mischung verwendet, die enthält, bezogen auf das Gesamtgewicht von (b1) und (b2),
   b1) 1 bis 20 Gew.-% mindestens eines Polyether-polyols mit einer mittleren Funktionalität von 3 bis 8, einer Hydroxylzahl von 200 bis 1000 und einem Gehalt an Alkaliionen von 150 bis 1200 ppm und
   b2) 99 bis 80 Gew.-% Ricinusöl und/oder mindestens eines Polyether-polyols mit einer Funktionalität von 2 bis 3, einer Hydroxylzahl von 90 bis 200 und einem Alkaliionengehalt von kleiner als 10 ppm.

Gegenstände der Erfindung sind ferner ein Verfahren zur Herstellung der transparenten, heißdampfsterilisierbaren, nicht zytotoxischen, im wesentlichen kompakten PU-Vergußmassen gemäß Anspruch 18 und die Verwendung der PU-Vergußmassen zum Einbetten von Hohlfasern aus vorzugsweise Polysulfonen oder Polycarbonaten in Dialysegeräte, zur Herstellung von medizinisch-technischen Artikeln sowie zur Bindung von Biokeramikbeschichtungen auf Endoprothesen nach Anspruch 17.

Die erfindungsgemäßen PU-Vergußmassen sind transparent, nicht zytotoxisch und besitzen nicht nur eine verbesserte Haftung zu anderen Werkstoffen, z. B. Polycarbonaten, bei erhöhten Temperaturen über einen längeren Zeitraum, sondern die Reaktionsmischungen lassen sich auch ohne Schaumbildung vergießen. Die PU-Vergußmassen sind bereits nach 2 Stunden schneidbar, härten jedoch nicht so stark nach, so daß sie auch nach mehr als 24 Stunden noch schneidfähig sind. Vorteilhaft ist ferner, daß die erfindungsgemäßen PU-Vergußmassen mit allen üblichen Hohlfaserarten, wie z. B. Cuprophan-, Polysulfon-, Polycarbonat- oder Cellulosefasern verarbeitbar sind und die Polycarbonate vor der Verarbeitung keiner Vorbehandlung durch Corona-Entladung zur Verbesserung der Haftfestigkeit bedürfen. Die PU-Vergußmassen sind gegen Percarbonsäuren stabil, so daß Formkörper aus solchen Pu-Vergußmassen mit Peressigsäure sterilisiert werden können.

Zu den zur Herstellung der erfindungsgemäßen PU-Vergußmassen verwendbaren Ausgangskomponenten (a) bis (c) ist folgendes auszuführen
a) Geeignete modifizierte Diphenylmethan-diisocyanate, üblicherweise abgekürzt MDI genannt, besitzen bei 23°C zweckmäßigerweise eine Viskosität von 100 bis 8000 m.Pa.s, vorzugsweise von 500 bis 3000 m.Pa.s und einen NCO-Gehalt von 29 bis 15 Gew.-%, vorzugsweise von 26 bis 15 Gew.-% und insbesondere von 24 bis 17 Gew.-%, bezogen auf Gesamtgewicht, und werden hergestellt nach an sich bekannten Verfahren durch Umsetzung von 4,4'-MDI oder 2,4'-MDI oder MDI-Isomerenmischungen, zweckmäßigerweise solchen, die, bezogen auf 100 Gew.-Teile, bestehen aus
   20 bis 90 Gew.-Teilen, vorzugsweise 50 bis 82 Gew.-Teilen 4,4'-MDI,
   80 bis 8 Gew.-Teilen, vorzugsweise 50 bis 8 Gew.-Teilen 2,4'-MDI und
   0 bis 5 Gew.-Teilen, vorzugsweise 0 bis 3 Gew.-Teilen 2,2'-MDI
   mit mindestens einem Polyoxypropylen-polyol, mindestens einem Polyoxypropylen-polyoxyethylen-polyol oder einer Mischung aus mindestens einem Polyoxypropylen-polyol und mindestens einem Polyoxypropylen-polyoxyethylen-polyol im Verhältnis von NCO-:OH-Gruppen von 2,5:1 bis 15:1, vorzugsweise von 5:1 bis 10:1 bei einer Temperatur von zweckmäßigerweise 50 bis 100°C, vorzugsweise 60 bis 90°C und in einer Reaktionszeit von 0,5 bis 3 Stunden, vorzugsweise von 1 bis 2 Stunden. Zur Modifizierung der MDI mit Urethangruppen durch partielle Reaktion von Isocyanatgruppen mit Hydroxylgruppen finden handelsübliche Polyoxypropylen-, Polyoxypropylen-polyoxyethylen- oder Polyoxyethylen-polyoxypropylen-polyole Verwendung, die üblicherweise einen Alkaligehalt von kleiner als 10 ppm, vorzugsweise von kleiner als 5 ppm besitzen. Neben den vorgenannten Polyoxyalkylen-polyolen können als alleinige Modifizierungsmittel oder in Mischungen mit Polyoxypropylen- und/oder Polyoxypropylen-polyoxyethylen-polyolen und/oder Polyoxyethylen-polyoxypropylen-polyolen auch Dipropylenglykol oder Ricinusöl oder Mischungen aus Dipropylenglykol und Ricinusöl verwendet werden.
   Zur Modifizierung der MDI beispielhaft genannt seien als Polyoxypropylen-polyole z. B. Polyoxypropylen-glykole mit einer Hydroxyzahl bis 400, Mischungen aus Dipropylen-glykol und Polyoxypropylen-glykol mit einer Hydroxylzahl bis 400, mit Glycerin, Trimethylolpropan oder einer Mischung aus Glycerin und Trimethylolpropan gestartete Polyoxypropylenpolyole mit einer Hydroxylzahl von 480 bis 210, mit Sucrose oder Sorbit oder Mischungen aus Sucrose und Sorbit gestartete Polyoxypropylen-polyole mit einer Hydroxyzahl von 250 bis 380, und als Polyoxypropylen-polyoxyethylen-polyole z. B. mit Sucrose oder Sorbit oder Mischungen aus Sucrose und Sorbit gestartete Polyoxypropylen-polyoxyethylen-polyole mit einem Gehalt an polymerisierten Ethylenoxideinheiten von 1 bis 80 Gew.-%, vorzugsweise 10 bis 50 Gew.-%, bezogen auf das Gesamtgewicht der Alkylenoxideinheiten, und einer Hydroxylzahl von 230 bis 500. Anstelle von Sucrose und/oder Sorbit können zur Herstellung der Polyoxypropylen- oder Polyoxypropylen-polyoxyethylen-polyole auch Mischungen aus den genannten Startermolekülen und mindestens einem Costarter aus der Gruppe Wasser, Propylenglykol, Glycerin und Trimethylolpropan Anwendung finden, wobei die Costarter zweckmäßigerweise nur in einer solchen Menge eingesetzt werden, daß die Funktionalität der resultierenden Polyoxyalkylenpolyole den Wert 4 nicht unterschreitet.
   Auf diese Weise modifizierte MDI werden beschrieben in der EP-A-0 393 545, Seite 4, Zeile 41 bis Seite 5, Zeile 8 und der EP-A-0 413 265, Seite 6, Zeile 31 bis Seite 7, Zeile 51, so daß diese Patentpublikationen als Bestandteil der Beschreibung zu betrachten sind.
   Als modifizierte MDI besonders bewährt haben sich jedoch, so daß diese bevorzugt Anwendung finden, Urethangruppen gebunden enthaltende NCO-Prepolymere mit einem NCO-Gehalt von 24 bis 15 Gew.-%, vorzugsweise von 22 bis 17 Gew.-%, die hergestellt werden durch Umsetzung von, bezogen auf das Gesamtgewicht, mindestens 85 Gew.-%, vorzugsweise 90 bis 96 Gew.-% eines NCO-gruppenhaltigen Prepolymeren mit einem NCO-Gehalt von 29 bis 21 Gew.-%, vorzugsweise von 26 bis 22 Gew.-%, das seinerseits hergestellt wird durch Umsetzung von 4,4'MDI mit Dipropylenglykol oder mindestens einem Polyoxypropylenglykol mit einer Hydroxyzahl bis 400, vorzugsweise von 50 bis 250 oder einer Mischung aus Dipropylenglykol und mindestens einem Polyoxypropylenglykol mit einer Hydroxylzahl bis 400 und maximal 15 Gew.-%, vorzugsweise 4 bis 10 Gew.-% Ricinusöl oder maximal 15 Gew.-% eines mit Glycerin, Trimethylolpropan oder einer Mischung aus Glycerin und Trimethylolpropan gestarteten Polyoxyalkylen-polyols mit einer Hydroxylzahl von 90 bis 200 und einem Alkaliionengehalt von kleiner als 10 ppm aus der Gruppe der Polyoxypropylen-, Polyoxypropylen-polyoxyethylen-und Polyoxyethylen-polyoxypropylen-polyole.
b) Als Verbindungen mit mindestens zwei reaktiven Wasserstoffatomen werden erfindungsgemäß Polyether-polyole mit einem im Vergleich zu handelsüblichen Produkten sehr hohen Alkaliionengehalt verwendet. Erfindungsgemäß verwendbar sind Polyether-polyole (b1) oder deren Mischungen mit einer mittleren Funktionalität von 3 bis 8, vorzugsweise von 3 bis 6, einer Hydroxylzahl von 200 bis 1000, vorzugsweise 230 bis 970 und insbesondere von 350 bis 750 und einem Gehalt an Alkaliionen, vorzugsweise von Kaliumionen, von 150 bis 1200 ppm, vorzugsweise von 200 bis 1000 ppm und insbesondere von 400 bis 600 ppm.
   Zur Herstellung der erfindungsgemäßen PU-Vergußmassen können als Aufbaukomponente (b) ausschließliche Polyether-polyole (bl) mit diesen Alkaliionengehalten verwendet werden. Als Aufbaukomponente (b) vorzüglich geeignet sind jedoch auch Mischungen, so daß diese vorzugsweise verwendet werden, die enthalten
   b1) mindestens ein Polyether-polyol (b1) mit der vorgenannten Spezifikation und
   b2) Ricinusöl oder mindestens ein Polyether-polyol mit einer Funktionalität von 2 bis 3, vorzugsweise von 2,3 bis 3, einer Hydroxylzahl von 90 bis 200, vorzugsweise von 120 bis 180 und insbesondere von 130 bis 160 und einem Alkaliionengehalt von kleiner als 10 ppm, vorzugsweise kleiner als 5 ppm oder Mischungen aus Ricinusöl und derartigen Polyether-polyolen (b2) mit geringem Alkaliionengehalt. Insbesondere bewährt haben sich Mischungen aus (b1) und (b2), die bezogen auf das Gesamtgewicht, enthalten oder vorzugsweise bestehen aus 1 bis 20 Gew.-%, vorzugsweise 1 bis 15 Gew.-% mindestens eines Polyether-polyols (bl) und 99 bis 80 Gew.-%, vorzugsweise 99 bis 85 Gew.-% Ricinusöl und/oder mindestens eines Polyether-polyols (b2).

   Als Polyether-polyole (bl) mit Alkaliionengehalt von 150 bis 1200 ppm können die an sich bekannten Polyether-polyole mit einer mittleren Funktionalität von 3 bis 8 und einer Hydroxylzahl von 200 bis 1000 verwendet werden mit der Maßgabe, daß die Polyether-polyole direkt mit dem erfindungsgemäß erforderlichen Alkaliionengehalt hergestellt werden oder vorzugsweise durch eine geeignete Methode der Alkaliionengehalt von handelsüblichen Polyether-polyolen, die üblicherweise einen Alkaliionengehalt von kleiner 10 ppm besitzen, erhöht wird. Zu diesem Zwecke können die Polyether-polyole mit wäßrigem Alkalihydroxid, vorzugsweise einer wäßrigen Kaliumhydroxidlösung, oder alkoholischen Alkalialkoholatlösungen, vorzugsweise alkoholischen Kaliumalkoholatlösungen, in den erforderlichen Mengen bei Raumtemperatur oder erhöhten Temperaturen, z.B. bei 20 bis 120°C behandelt werden. Danach wird das zugesetzte und gebildete Wasser bzw. der Alkohol bei einer Temperatur im Bereich von 70 bis 110°C, gegebenenfalls unter vermindertem Druck, z.B. von 0,01 bis 1 mbar, abdestilliert.
   Als Polyether-polyole (bl) vorzüglich bewährt haben sich und daher vorzugsweise verwendet werden z.B. mit Trimethylolpropan gestartete Polyoxyethylen-polyole mit einer Hydroxylzahl im Bereich von 632 bis 970 und einem Kaliumionengehalt im Bereich von 400 bis 600 ppm und mit Glycerin oder Trimethylolpropan oder einer Glycerin-Trimethylolpropanmischung gestartete Polyoxypropylenpolyole mit einer Hydroxylzahl im Bereich von 210 bis 480 und einem Kaliumionengehalt im Bereich von 400 bis 600 ppm. Als alkalireiche Polyether-polyole eignen sich ferner z.B. Polyoxypropylen-polyole mit einer mittleren Funktionalität von 4 bis 8, vorzugsweise 4 bis 6 und einer Hydroxylzahl von 230 bis 500, vorzugsweise von 250 bis 380, die erhalten werden unter Verwendung von Sucrose oder vorzugsweise Sorbit oder Mischungen aus Sucrose und Sorbit als Startermoleküle, wobei als Costarter zusätzlich Wasser, Propylenglykol, Glycerin oder Mischungen aus mindestens zwei der genannten Costarter mitverwendet werden können, mit der Maßgabe, daß die Polyether-polyole einen Alkaliionengehalt, vorzugsweise Kaliumionengehalt, von 200 bis 1000 ppm, vorzugsweise 400 bis 700 ppm besitzen. In Betracht kommen ferner Polyoxypropylen- und/oder Polyoxyethylen-polyole mit einem Alkaliionengehalt von 150 bis 800 ppm und einer Hydroxylzahl von 450 bis 750, die erhalten werden können durch Umsetzung von Pentaerythrit oder einer Mischung aus Pentaerythrit und Glycerin und/oder Trimethylolpropan, zweckmäßigerweise in einem Molverhältnis von Pentaerythrit zu Glycerin und/oder Trimethylolpropan von 1:1, mit 1,2-Propylenoxid oder Ethylenoxid. Als alkalireiche Polyether-polyole verwendbar sind auch Polyoxypropylen-polyoxyethylen-polyole, die z.B. erhalten werden durch Polyaddition von 1,2-Propylenoxid und Ethylenoxid in einem Molverhältnis von 1:1 bis 1:8, vorzugsweise von 1:1 bis 1:3 an Glycerin, Trimethylpropan oder eine Mischung aus Glycerin und Trimethylolpropan als Startermoleküle, mit einer Hydroxylzahl von 350 bis 950, vorzugsweise von 380 bis 600 und einem Alkaliionengehalt, vorzugsweise Kaliumionengehalt, von 200 bis 800 ppm, vorzugsweise von 400 bis 600 ppm, oder durch Polyaddition von 1,2-Propylenoxid und Ethylenoxid in einem Molverhältnis von 1:1 bis 1:8, vorzugsweise von 1:1 bis 1:3 an Sucrose oder vorzugsweise Sorbit oder Mischungen aus Sucrose und Sorbit als Startermoleküle, mit einer Hydroxylzahl von 200 bis 500, vorzugsweise von 230 bis 300 und einem Alkaliionengehalt, vorzugsweise Kaliumionengehalt von 200 bis 800 ppm, vorzugsweise von 400 bis 600 ppm. Die beispielhaft genannten Polyether-polyole (b1) können einzeln oder in Form von Mischungen verwendet werden.
   Als Aufbaukomponente (b2) der Mischung aus (b1) und (b2) kann, wie bereits ausgeführt wurde, Ricinusöl verwendet werden. Geeignet sind jedoch auch Polyether-polyole mit einem Alkaligehalt kleiner als 10 ppm, z.B. Polyoxypropylen-polyole, Polyoxyethylen-polyoxypropylen-polyole mit einem Gehalt an endständig polymerisierten Propylenoxideinheiten von 1 bis 15 Gew.-%, bezogen auf das Gesamtgewicht der Alkylenoxideinheiten, oder vorzugsweise Polyoxypropylen-polyoxyethylen-polyole mit einem Gehalt an polymerisierten Ethylenoxideinheiten von 60 bis 90 Gew.-%, bezogen auf das Gesamtgewicht der Alkylenoxideinheiten. Vorzugsweise verwendet werden trifunktionelle, insbesondere mit Glycerin oder Trimethylolpropan gestartete Polyether-polyole z.B. Polyoxypropylen-, Polyoxypropylen-polyoxyethylen-polyole oder Polyoxyethylen-polyoxypropylen-polyole, mit einer Hydroxylzahl von 120 bis 180 und einem Alkaliionengehalt von kleiner als 5 ppm.
   Die erfindungsgemäßen PU-Vergußmassen können ohne die Mitverwendung von mindestens trifunktionellen Vernetzungsmitteln hergestellt werden. Sofern jedoch Vernetzungsmittel zur Modifizierung der mechanischen Eigenschaften eingesetzt werden, finden zweckmäßigerweise hydroxylgruppenhaltige Vernetzungsmittel mit einer Funktionalität von 3 bis 8, vorzugsweise 3 bis 4 Verwendung. Als geeignete Vernetzungsmittel seien beispielhaft genannt: 3- und höherwertige Alkohole, wie z.B. Glycerin, Trimethylolpropan, Pentaerythrit, 2, 2, 6, 6-Tetrahydroxymethyl-4-oxaheptandiol-1, 7(Di-pentaerythrit), Tripentaerythrit, 3,3,7,7-Tetrahydroxymethyl-5-oxanonane(Di-trimethylolpropan) und Sorbit und die mit diesen Alkoholen gestarteten niedermolekularen Polyoxypropylen-, Polyoxyethylen- oder Polyoxypropylen-polyoxyethylen-polyolen.
c) Die erfindungsgemäßen PU-Vergußmassen können in Abwesenheit oder Gegenwart von Katalysatoren hergestellt werden. Die Herstellung der PU-Vergußmassen findet jedoch vorzugsweise in Gegenwart von Katalysatoren statt, die die Reaktion der modifizierten Diphenylmethan-diisocyanate (a) mit den Hydroxylgruppen enthaltenden Verbindungen mit mindestens zwei reaktiven Wasserstoffatomen (b) stark beschleunigen. Als Katalysatoren in Betracht kommen organische Metallverbindungen, vorzugsweise organische Zinnverbindungen, wie z.B. Zinn-(II)-salze von organischen Carbonsäuren, z.B. Zinn-(II)-diacetat, Zinn-(II)-dioctoat, Zinn-(II)-diethylhexoat und Zinn-(II)-dilaurat und die Dialkylzinn-(IV)-salze von organischen Carbonsäuren, z.B. Dibutyl-zinndiacetat, Dibutylzinn-dilaurat, Dibutylzinn-maleat und Dioctylzinndiacetat. Derartige Katalysatoren werden z.B. in der DE-A-3 048 529 beschrieben. Als gut geeignet haben sich auch Dialkylzinn(IV)-mercaptoverbindungen erwiesen, wie z.B. Bislaurylzinn(IV)-dimercaptid, und Verbindungen der allgemeinen Formeln R₂Sn(SR'-O-CO-R")₂ oder R₂Sn(SR'-CO-OR")₂, in denen R einen Alkylrest mit mindestens 8 Kohlenstoffatomen, R' einen Alkylenrest mit mindestens 2 Kohlenstoffatomen und R" einen Alkylrest mit mindestens 4 Kohlenstoffatomen bedeuten. Als Katalysatoren dieser Art, die beispielsweise in der DD-A-218 668 beschrieben werden, seien beispielhaft genannt: Dioctylzinn-bis(thioethylenglykol-2-ethylhexoat), Dioctylzinn-bis(thioethylenglykollaurat), Dioctylzinn-bis(thiolatoessigsäure-2-ethylhexylester, Dioctylzinn-bis(thiolatoessigsäurehexylester) und Dioctylzinn-bis(thiolatoessigsäurelaurylester. Als Katalysatoren sehr gut bewährt haben sich ferner Organozinnverbindungen mit Zinn-Sauerstoff- oder Zinn-Schwefel-Bindungen, wie sie beispielsweise in der DD-A-255 535 beschrieben werden, der allgemeinen Formeln (R₃Sn)₂O, R₂SnS, (R₃Sn)₂S, R₂Sn(SR')₂ oder RSn(SR')₃, in denen R und R' Alkylgruppen darstellen, die 4 bis 8 Kohlenstoffatome bei R sowie 4 bis 12 Kohlenstoffatome bei R' enthalten und R' außerdem die Reste -R"COOR"' und -R"OCOR‴ bedeuten kann, in denen R" Alkylgruppen mit 1 bis 6 Kohlenstoffatomen und R‴ Alkylengruppen mit 4 bis 12 Kohlenstoffatomen sind. Als Beispiele hierfür seien genannt: Bis(tributylzinn)oxid, Dibutylzinnsulfid, Dioctylzinnsulfid, Bis(tributylzinn)sulfid, Dibutylzinn-bis(thioglykolsäure-2-ethylhexylester), Dioctylzinn-bis(thioglykolsäure-2-ethylhexylester), Octylzinn-tris(thioglykolsäure-2-ethyl-hexylester), Dioctylzinn-bis(thioethylenglykol-2-ethyl-hexoat) und Dibutylzinn-bis(thioethylenglykollaurat). Als Katalysatoren vorzugsweise verwendet werden Mono-n-octylzinn-(2-ethylhexyl-thioglykolat), Di-n-octylzinn-bis-(2-ethylhexyl-thioglykolat) und Dibutylzinn-dilaurat.
   Die organischen Metallverbindungen können als Katalysatoren einzeln oder in Form von Katalysatorkombinationen eingesetzt werden. Als äußerst vorteilhaft haben sich Kombinationen erwiesen, die bestehen aus, bezogen auf das Gesamtgewicht, 1 bis 99 Gew.-% Mono-n-octylzinn-(2-ethylhexylthioglykolat) und 99 bis 1 Gew.-% Di-n-octylzinn-bis(2-ethylhexylthioglykolat) oder 94 Gew.-% Di-n-octylzinn-bis-(2-ethylhexylthioglykolat) und 6 Gew.-% Mono-n-octyl-zinn-tris-(2-ethylhexylthioglykolat).
   Die Katalysatoren werden üblicherweise in einer Menge von 0,001 bis 0,2 Gew.-Teilen, vorzugsweise 0,005 bis 0,015 Gew.-Teilen pro 100 Gew.-Teile der Aufbaukomponente (b) verwendet.

Zur Herstellung der erfindungsgemäßen PU-Vergußmassen werden die modifizierten MDI (a) und Verbindungen mit mindestens zwei reaktiven Wasserstoffatomen (b), vorzugsweise in Gegenwart der Katalysatoren (c) in solchen Mengen zur Umsetzung gebracht, daß das Äquivalenz-Verhältnis von NCO-Gruppen der modifizierten MDI (a) zur Summe der reaktiven Wasserstoffatome der Komponente (b) 0,9 bis 1,3:1, vorzugsweise 0,95 bis 1,2:1 und insbesondere 1,0 bis 1,1:1 beträgt. Hierzu werden die im wesentlichen vollständig entgasten Ausgangskomponenten bei Temperaturen von zweckmäßigerweise 18 bis 70°C, vorzugsweise von 22 bis 60°C intensiv gemischt. Die Reaktionsmischungen, die einen Gesamtgehalt an Alkaliionen, vorzugsweise Kaliumionen, von vorzugsweise 8 bis 110 ppm, insbesondere 15 bis 50 ppm aufweisen, werden in ein geeignetes Formwerkzeug eingebracht und über einen Zeitraum von 0,3 bis 4 Stunden, vorzugsweise von 1 bis 3 Stunden aushärten gelassen.

Wie bereits dargelegt wurde, finden die transparenten, heißdampfsterilisierbaren, nicht zytotoxischen, im wesentlichen kompakten, PU-Vergußmassen, insbesondere Verwendung zum Einbetten von Hohlfasern, wie z.B. von Cuprophan- und vorzugsweise von Polysulfon-, Polycarbonatfasern oder Cellulosehohlfasern in Dialysatoren, wobei die Dialysegeräte, insbesondere die Hülse für das Dialysefilter, zweckmäßigerweise aus einem Polycarbonat auf Basis von Bisphenol A besteht.

Die erfindungsgemäßen PU-Vergußmassen eignen sich ferner zur Herstellung von medizinisch-technischen Artikeln und zur Bindung von Biokeramikbeschichtungen auf Endoprothesen.

Die PU-Vergußmassen sind nicht zytotoxisch, transparent, zeigen keine Wechselwirkung mit den Hohlfasern, besitzen eine starke Haftung zum Polycarbonat und sind ohne Zerstörung der eingebetten Hohlfasern gut zerschneidbar.

### Beispiel 1

### Herstellung des modifizierten MDI

93,99 Gew.-Teile eines Urethangruppen enthaltenden MDI-Prepolymeren mit einem NCO-Gehalt von 23 Gew.-%, das hergestellt wurde durch Umsetzung von 4,4'-MDI mit einer Mischung aus Dipropylen-glykol und einem Polyoxypropylen-glykol mit einer Hydroxylzahl 250 und einem Kaliumionengehalt von 3 ppm im Gewichtsverhältnis von 1:0,6,
wurden auf 60°C unter Rühren erwärmt und innerhalb von 30 Minuten, tropfenweise, mit
6,01 Gew.-Teilen Ricinusöle, das einen Wassergehalt von
0,03 Gew.-% aufwies, gemischt. Die Reaktionsmischung wurde auf 80°C erwärmt und bei dieser Temperatur die Reaktion in einer Stunde zu Ende geführt.

Die erhaltende, mit Urethangruppen modifizierte MDI-Mischung besaß einen NCO-Gehalt von 20,5 Gew.-% und eine Viskosität bei 25°C von 2100 m.Pa.s.

### Beispiel 2

### Herstellung der PU-Vergußmasse

### A-Komponente:

### Mischung aus

5 Gew.-Teilen eines Polyether-polyols mit einer Hydroxylzahl von 931 und einem Kaliumionengehalt von 508 ppm, hergestellt durch Addition von Ethylenoxid an Trimethylolpropan als Startermolekül in Gegenwart von Kaliumhydroxid als Katalysator,
94,95 Gew.-Teilen Ricinusöl und
0,05 Gew.-Teilen einer Katalysatorkombination, bestehend, bezogen auf das Gesamtgewicht, aus
   94 Gew.-% Di-n-octylzinn-bis(2-ethylhexylthioglykolat) und
   6 Gew.-% Mono-n-octylzinn-tris-(2-ethylhexylthioglykolat).

### B-Komponente:

### modifizierte MDI-Mischung gemäß Beispiel 1

100 Gew.-Teile der A-Komponente und
77,7 Gew.-Teile der B-Komponente,
entsprechend einem NCO-Index von 105, wurden bei 23°C intensiv gemischt; die Reaktionsmischung in ein Formwerkzeug eingegossen und aushärten gelassen.

Die Reaktionsmischung besaß einen Kaliumionengehalt von 25,4 ppm und zeigte eine Gelzeit von 350 Sekunden.

Der Formkörper besaß eine Härte von 96,5 Shore-A nach 24-stündiger Lagerung bei 23°C.

### Beispiel 3

A-Komponente: analog Beispiel 2,
wobei jedoch die Ausgangsstoffe in folgenden Mengen verwendet wurden
5,2 Gew.-Teile des Polyether-polyols mit der Hydroxylzahl von 931 und einem Kaliumionengehalt von 508 ppm,
94,7 Gew.-Teile des Ricinusöls und
0,1 Gew.-Teile der Katalysatorkombination.

### B-Komponente: modifizierte MDI-Mischung gemäß Beispiel 1

100 Gew.-Teile der A-Komponente mit einer Temperatur von 40°C und 77,7 Gew.-Teile der B-Komponente mit einer Temperatur von 40°C, entsprechend einem NCO-Index von 105, wurden auf einer Niederdruckmaschine gemischt und die Reaktionsmischung, die einen Kaliumionengehalt von 26,4 ppm besap, in eine Zentrifuge mit einer Temperatur von 40°C eingebracht und 5 Minuten geschleudert.

Der nach dem Schleuderverfahren hergestellte Formkörper war unmittelbar nach der Entformung blasenfrei und trocken und nach 2 Stunden schneidfähig.

Aus Polycarbonathülsen, Polysulfonhohlfasern und den Pu-Vergußmassen hergestellt Dialysatoren konnten bei 121°C mindestens 6 mal heißdampfsterilisiert werden, ohne daß eine Beschädigung am Formkörper auftrat. Die zytotoxische Unbedenklichkeitsprüfung nach den Richtlinien "Guide line ISO/TR 7405-1984(E)" publiziert von der International Organization for Standardization: "Biological Evaluation of Dental Materials sowie die Vornorm DIN V 13930, Sept. 1990" "Biologische Prüfungen von Dentalwerkstoffen" wurde bestanden.

Ferner ergab eine 10 tägige Lagerung des Formkörpers bei 23°C in 7 gew.-%iger wäßriger Peressigsäure keinen Abfall des Peressigsäuregehalts.

### Beispiel 4

### Herstellung des modifizierten MDI

2781,85 Gew.-Teile eines Urethangruppen enthaltenden MDI-Prepolymeren mit einem NCO-Gehalt von 23 Gew.-%, das hergestellt wurde durch Umsetzung von 4,4'-MDI mit einer Mischung aus Dipropylen-glykol und einem Polyoxypropylenglykol mit einer Hydroxylzahl von 250 und einem Kaliumionengehalt von 3 ppm im Gewichtsverhältnis von 1:0,6 wurden auf 60°C unter Rühren erwärmt und innerhalb von 30 Minuten, tropfenweise, mit
219,83 Gew.-Teilen eines mit Trimethylolpropan gestarteten Polyoxypropylen-polyols mit einer OH-Zahl von 148,5 und einem Kaliumionengehalt von 3 ppm, gemischt. Die Reaktionsmischung wurde auf 80°C erwärmt und bei dieser Temperatur die Reaktion in einer Stunde zu Ende geführt.

Die erhaltene, mit Urethangruppen modifizierte MDI-Mischung besaß einen NCO-Gehalt von 20,3 Gew.-% und eine Viskosität bei 25°C von 3324 m.Pas.

### Beispiel 5

### Herstellung der PU-Vergußmasse

### A-Komponente

| Mischung aus | |
|---|---|
| 7,3 | Gew.-Teilen eines Polyether-polyols mit einer Hydroxylzahl von 923 und einem Kaliumionengehalt von 534 ppm, hergestellt durch Addition von Ethylenoxid an Trimethylolpropan als Startermolekül in Gegenwart von Kaliumhydroxid als Katalysator, |
| 92,65 | Gew.-Teilen eines mit Trimethylolpropan gestarteten Polyoxypropylen-polyols mit einer OH-Zahl von 148,5 und einem Kaliumionengehalt von 3 ppm und |
| 0,05 | Gew.-Teilen einer Katalysatorkombination, bestehend, bezogen auf das Gesamtgewicht, aus 94 Gew.-% Di-n-octylzinn-bis(2-ethylhexylthioglykolat) und 6 Gew.-% Mono-n-octylzinn-tris(2-ethylhexyl-thioglykolat) |

### B-Komponente: mit Urethangruppen modifizierte MDI-Mischung gemäß Beispiel 4

| | |
|---|---|
| 100 | Gew.-Teile der A-Komponente und |
| 79,18 | Gew.-Teile der B-Komponente, entsprechend einem NCO-Index von 105, wurden bei 23°C intensiv vermischt; die Reaktionsmischung wurde in einen Polystyrolbecher eingegossen und aushärten gelassen. |

Die Reaktionsmischung besaß einen Kaliumionengehalt von 39 ppm und zeigte eine Gelzeit von 203 Sekunden.

Der hergestellte Formkörper besap nach einer 24 stündigen Lagerung bei 23°C eine Härte von 98 Shore-A. Aufgrund der äußerst starken Haftung zwischen Polystyrol und der PU-Vergußmasse konnten diese nicht wieder getrennt werden. Eine derartig starke Haftung konnte zwischen Polystyrol und konventionellen PU-Polyether-polyolvergußmassen nicht ermittelt werden.

### Beispiel 6

### Herstellung einer mit Urethangruppen modifizierten MDI-Mischung

Man verfuhr analog den Angaben des Beispiels 4, verwendete jedoch anstelle von 219,83 Gew.-Teilen des Polyoxypropylen-polyols 218,15 Gew.-Teile eines mit Trimethylolpropan gestarteten Polyoxypropylen (80 Gew.-%) -polyoxyethylen (20 Gew.-%) -polyols mit einer Hydroxylzahl von 152 und einem Kaliumionengehalt von 3 ppm.

Die erhaltene, mit Urethangruppen modifizierte MDI-Mischung besaß einen NCO-Gehalt von 20,2 Gew.-% und eine Viskosität bei 25°C von 3266 m.Pas.

### Beispiel 7

### Herstellung der PU-Vergußmasse

### A-Komponente:

| Mischung aus | |
|---|---|
| 7 | Gew.-Teilen eines Polyether-polyols mit einer Hydroxylzahl von 923 und einem Kaliumionengehalt von 534 ppm, hergestellt durch Addition von Ethylenoxid an Trimethylolpropan als Startermolekül in Gegenwart von Kaliumhydroxid als Katalysator, |
| 92,95 | Gew.-Teilen eines mit Trimethylolpropan gestarteten Polyoxypropylen (80 Gew.-%) -polyoxyethylen (20 Gew.-%)-polyols mit einer Hydroxylzahl von 152 und einem Kaliumionengehalt von 3 ppm und |
| 0,05 | Gew.-Teilen einer Katalysatorkombination bestehend, bezogen auf das Gesamtgewicht, aus 94 Gew.-% Di-n-octylzinn-bis(2-ethylhexyl-thioglykolat) und 6 Gew.-% Mono-n-octylzinn-tris-(2-ethylhexyl-thioglykolat) |

### B-Komponente: mit Urethangruppen modifizierte MDI-Mischung gemäß Beispiel 6

| | |
|---|---|
| 100 | Gew.-Teile der A-Komponente und |
| 80,01 | Gew.-Teile der B-Komponente, entsprechend einem NCO-Index von 105, wurden bei 23°C intensiv gemischt; die Reaktionsmischung wurde in einen Polystyrolbecher eingegossen und aushärten gelassen. |

Die Reaktionsmischung besaß einen Kaliumgehalt von 37,4 ppm und zeigte eine Gelzeit von 124 Sekunden. Der Formkörper besaß eine Härte von 96 Shore-A nach einer 24 stündigen Lagerung bei 23°C. Die Haftung der PU-Vergupmasse an das Polystyrol war so stark, daß sich die Komponenten nicht wieder trennen ließen. Eine 10 tägige Lagerung des Formkörpers bei 23°C in 7 gew.-%iger wäßriger Peressigsäure ergab keinen Abfall des Peressigsäuregehalts.

### Beispiel 8

### Herstellung einer PU-Vergußmasse

### A-Komponente:

| Mischung aus | |
|---|---|
| 11,1 | Gew.-Teilen eines Polyether-polyols mit einer Hydroxylzahl von 657 und einem Kaliumionengehalt von 482 ppm, hergestellt durch Addition an einer Startermischung aus Pentaerythrit und Trimethylolpropan im Molverhältnis von 1:1 von 1,2-Propylenoxid und danach Ethylenoxid im Gewichtsverhältnis von 47:53 Gew.-%, bezogen auf das Alkylenoxidgewicht, |
| 88,85 | Gew.-Teilen eines mit Trimethylolpropan gestarteten Polyoxypropylen-polyols mit einer Hydroxylzahl von 148,5 und einem Kaliumionengehalt von 3 ppm und |
| 0,05 | Gew.-Teilen einer Katalysatorkombination, bestehend aus, bezogen auf das Gesamtgewicht, 94 Gew.-% Di-n-octylzinn-bis(2-ethylhexyl-thiogykolat) und 6 Gew.-% Mono-n-octylzinn-tris(2-ethylhexylthio-glykolat). |

### B-Komponente: mit Urethangruppen modifizierte MDI-Mischung mit einem NCO-Gehalt von 20,23 Gew.-%, hergestellt analog den Angaben des Beispiels 4.

100 Gew.-Teile der A-Komponente und
79,14 Gew.-Teile der B-Komponente, entsprechend einem NCO-Index von 105, wurden bei 23°C intensiv vermischt; die Reaktionsmischung wurde in einen Polystyrolbecher eingegossen und aushärten gelassen.

Die Reaktionsmischung besaß einen Kaliumionengehalt von 53,5 ppm und zeigte eine Gelzeit von 177 Sekunden.

Der Formkörper besaß eine Härte von 96 Shore-A nach 24 stündiger Lagerung bei 23°C. Die Haftung zwischen Polystyrol und der PU-Vergußmasse war hervorragend.

### Beispiel 9

### Herstellung einer PU-Vergußmasse

### A-Komponente:

| Mischung aus | |
|---|---|
| 7 | Gew.-Teile eines Polyether-polyols mit einer Hydroxylzahl von 923 und einem Kaliumionengehalt von 534 ppm, hergestellt durch Addition von Ethylenoxid an Trimethylolpropan in Gegenwart von Kaliumhydroxid als Katalysator, |
| 92,992 | Gew.-Teilen eines mit Trimethylolpropan gestarteten Polyoxypropylen (80 Gew.-%) -polyoxyethylen (20 Gew.-%) -polyols mit einer Hydroxylzahl von 152 und einem Kaliumgehalt von 3 ppm und |
| 0,008 | Gew.-Teilen Dibutylzinndilaurat. |

### B-Komponente: mit Urethangruppen modifizierte MDI-Mischung gemäß Beispiel 6.

100 Gew.-Teile der A-Komponente und
80,03 Gew.-Teile der B-Komponente, entsprechend einem NCO-Index von 105, wurden bei 23°C intensiv gemischt. Die Reaktionsmischung wurde in einen Polystyrolbecher eingegossen und aushärten gelassen.

Die Reaktionsmischung besap einen Kaliumionengehalt von 37,4 ppm und zeigte eine Gelzeit von 234 Sekunden.

Der Formkörper besaß nach einer 24 stündigen Lagerung bei 23°C eine Härte von 96 Shore-A. Die Haftung zwischen der PU-Vergußmasse und dem Polystyrol war ausgezeichnet.

### Beispiel 10

### Herstellung einer PU-Vergußmasse

### A-Komponente:

| Mischung aus | |
|---|---|
| 7 | Gew.-Teile eines Polyether-polyols mit einer Hydroxylzahl von 923 und einem Kaliumionengehalt von 534 ppm, hergestellt durch Addition von Ethylenoxid an Trimethylolpropan in Gegenwart von Kaliumhydroxid als Katalysator und |
| 93 | Gew.-Teilen eines mit Trimethylolpropan gestarteten Polyoxypropylen (80 Gew.-%) -polyoxyethylen (20 Gew.-%)-polyols mit einer Hydroxylzahl von 152 und einem Kaliumgehalt von 3 ppm. |

### B-Komponente: mit Urethangruppen modifizierte MDI-Mischung gemäß Beispiel 6.

100 Gew.-Teile der A-Komponente und
80,04 Gew.-Teile der B-Komponente, entsprechend einem NCO-Index von 105, wurden bei 23°C intensiv gemischt. Die Reaktionsmischung wurde in einen Polystyrolbecher eingegossen und aushärten gelassen.

Die Reaktionsmischung besaß einen Kaliumionengehalt von 37,4 ppm und zeigte eine Gelzeit von 520 Sekunden.

Der Formkörper besaß nach einer 24 stündigen Lagerung bei 23°C eine Härte von 91 Shore-A. Die Haftung zwischen der PU-Vergußmasse und dem Polystyrol war befriedigend.

### Beispiel 11

### Herstellung des modifizierten MDI

91,8 Gew.-Teile eines Urethangruppen enthaltenden MDI-Prepolymeren mit einem NCO-Gehalt von 23 Gew.-%, das hergestellt wurde durch Umsetzung von 4,4'-MDI mit einer Mischung aus Dipropylen-glykol und einem Polyoxypropylen-glykol mit einer Hydroxylzahl von 250 und einem Kaliumionengehalt von 3 ppm im Gewichtsverhältnis von 1:0,6,
wurden auf 60°C unter Rühren erwärmt und innerhalb von 30 Minuten, tropfenweise, mit
8,2 Gew.-Teilen eines mit Trimethylolpropan gestarteten Polyoxyethylen (95 Gew.-%) -polyoxypropylen (5 Gew.-%) -polyols mit einer Hydroxylzahl von 100 und einem Kaliumionengehalt von 3 ppm gemischt. Die Reaktionsmischung wurde auf 80°C erwärmt und bei dieser Temperatur die Reaktion in einer Stunde zu Ende geführt.

Die erhaltende, mit Urethangruppen modifizierte MDI-Mischung besaß einen NCO-Gehalt von 20,15 Gew.-% und eine Viskosität bei 25°C von 2414 m.Pa.s.

### Beispiel 12

### Herstellung der PU-Vergußmasse

### A-Komponente: analog Beispiel 10

### B-Komponente: modifizierte MDI-Mischung nach Beispiel 11

100 Gew.-Teile der A-Komponente und
80,75 Gew.-Teile der B-Komponente, entsprechend einem NCO-Index von 105, wurden bei 23°C intensiv gemischt. Die Reaktionsmischung wurde in einen Polystyrolbecher eingegossen und aushärten gelassen.

Die Reaktionsmischung besaß einen Kaliumionengehalt von 69,4 ppm und zeigte eine Gelzeit von 173 Sekunden.

Der Formkörper besaß nach einer 24 stündigen Lagerung bei 23°C eine Härte von 75 Shore-A. Die Haftung zwischen der PU-Vergußmasse und dem Polystyrol war befriedigend.

### Beispiel 13

### Herstellung der PU-Vergußmasse

### A-Komponente: analog Beispiel 5, jedoch mit einem Gehalt der Katalysatorkombination von 0,02 Gew.-Teilen.

### B-Komponente: analog Beispiel 4

100 Gew.-Teile der A-Komponente und
79,18 Gew.-Teile der B-Komponente, entsprechend einem NCO-Index von 105, wurden, jeweils entgast und auf 40°C erwärmt, mit Hilfe einer Niederdruckmaschine gemischt und die Reaktionsmischung in eine Zentrifuge mit einer Temperatur von 40°C eingebracht und 5 Minuten geschleudert.

Der nach dem Schleuderverfahren hergestellte Formkörper war unmittelbar nach der Entformung blasenfrei und trocken und nach 2 Stunden schneidfähig.

Aus Polycarbonathülsen, Polysulfonhohlfasern und den Pu-Vergußmassen hergestellt Dialysatoren konnten bei 121°C mindestens 6 mal heißdampfsterilisiert werden, ohne daß eine Beschädigung am Formkörper auftrat. Ferner ergab eine 10 tägige Lagerung des Formkörpers bei 23°C in 7 gew.-%iger wäßriger Peressigsäure keinen Abfall des Peressigsäuregehalts. Die zytotoxische Unbedenklichkeitsprüfung nach den Richtlinien Guide line ISO/TR 7405-1984(E) wurde bestanden.

## Patentansprüche

1. Transparente, heißdampfsterilisierbare, nicht zytotoxische, im wesentlichen kompakte Polyurethan-Vergußmassen, hergestellt durch Umsetzung von
a) modifizierten Diphenylmethan-diisocyanaten mit einer Viskosität bei 23-C von 100 bis 8000 mPas mit
b) Verbindungen mit mindestens zwei reaktiven Wasserstoffatomen
in Gegenwart oder Abwesenheit von
c) Katalysatoren
dadurch gekennzeichnet, daß man als Komponente (b) eine Mischung verwendet, die enthält, bezogen auf das Gesamtgewicht von (b1) und (b2),
b1) 1 bis 20 Gew.-% mindestens eines Polyether-polyols mit einer mittleren Funktionalität von 3 bis 8, einer Hydroxylzahl von 200 bis 1000 und einem Gehalt an Alkaliionen von 150 bis 1200 ppm und
b2) 99 bis 80 Gew.-% Ricinusöl und/oder mindestens eines Polyether-polyols mit einer Funktionalität von 2 bis 3, einer Hydroxylzahl von 90 bis 200 und einem Alkaliionengehalt von kleiner als 10 ppm.

2. Transparente, heißdampfsterilisierbare, nicht zytotoxische, im wesentlichen kompakte Polyurethan-Vergußmassen nach Anspruch 1, dadurch gekennzeichnet, daß die Mischungskomponente (b2) aus Ricinusöl besteht.

3. Transparente, heißdampfsterilisierbare, nicht zytotoxische, im wesentlichen kompakte Polyurethan-Vergußmassen nach Anspruch 1, dadurch gekennzeichnet, daß die Mischungskomponente (b) aus einem trifunktionellen Polyether-polyol mit einer Hydroxylzahl von 120 bis 180 und einem Alkaliionengehalt von kleiner als 5 ppm besteht.

4. Transparente, heißdampfsterilisierbare, nicht zytotoxische, im wesentlichen kompakte Polyurethan-Vergußmassen nach An- / spruch 1, dadurch gekennzeichnet, daß die Alkaliionen in (bl) aus Kaliumionen bestehen.

5. Transparente, heißdampfsterilisierbare, nicht zytotoxische, im wesentlichen kompakte Polyurethan-Vergußmassen nach Anspruch 1, dadurch gekennzeichnet, daß das Polyether-polyol (b) ein mit Trimethylolpropan gestartetes Polyoxyethylenpolyol mit einer Hydroxylzahl im Bereich von 632 bis 970 und einem Kaliumionengehalt im Bereich von 400 bis 600 ppm ist.

6. Transparente, heißdampfsterilisierbare, nicht zytotoxische, im wesentlichen kompakte Polyurethan-Vergußmassen nach Anspruch 1, dadurch gekennzeichnet, daß das Polyether-polyol (b1) ein mit Glycerin und/oder Trimethylolpropan gestartetes Polyoxypropylen-polyol mit einer Hydroxylzahl im Bereich von 210 bis 480 und einem Kaliumionengehalt im Bereich von 400 bis 600 ppm ist.

7. Transparente, heißdampfsterilisierbare, nicht zytotoxische, im wesentlichen kompakte Polyurethan-Vergußmassen nach Anspruch 1, dadurch gekennzeichnet, daß das Polyetherpolyol (bl) besteht aus mindestens einem Polyoxypropylen-polyol mit einer mittleren Funktionalität von 4 bis 8 und einer Hydroxylzahl von 230 bis 500, erhalten unter Verwendung von Sucrose oder Sorbit oder Mischungen aus Sucrose und Sorbit als Startermoleküle, wobei als Costarter zusätzlich Wasser, Propylenglykol, Glycerin oder Mischungen aus mindestens zwei der genannten Costarter mitverwendet werden können, mit der Maßgabe, daß die Polyether-polyole einen Alkaliionengehalt von 400 bis 700 ppm besitzen.

8. Transparente, heißdampfsterilisierbare, nicht zytotoxische, im wesentlichen kompakte Polyurethan-Vergußmassen nach Anspruch 1, dadurch gekennzeichnet, daß das Polyetherpolyol (bl) ein Polyoxypropylen- oder Polyoxyethylen-polyol oder eine Mischung dieser Polyoxyalkylen-polyole mit einer Hydroxylzahl von 450 bis 750 ist, erhalten unter Verwendung von Pentaerythrit oder einer Mischung aus Pentaerythrit und Glycerin und/oder Trimethylpolypropan als Startermoleküle, mit der Maßgabe, daß die Polyether-polyole einen Alkaliionengehalt von 150 bis 800 ppm besitzen.

9. Transparente, heißdampfsterilisierbare, nicht zytotoxische, im wesentlichen kompakte Polyurethan-Vergußmassen nach Anspruch 1, dadurch gekennzeichnet, daß man als modifizierte Diphenylmethan-diisocyanate (a) Urethangruppen gebunden enthaltende NCO-Prepolymere mit einem NCO-Gehalt von 24 bis 15 Gew.-% verwendet, die hergestellt werden durch Umsetzung von, bezogen auf das Gesamtgewicht, mindestens 85 Gew.-% eines NCO-gruppenhaltigen Prepolymeren mit einen NCO-Gehalt von 29 bis 21 Gew.-%, das seinerseits erhalten wird durch Umsetzung von 4,4'-Diphenylmethan-diisocyanat mit Dipropylenglykol oder mindestens einem Polyoxypropylen-glykol mit einer Hydroxyzahl bis 400 oder einer Mischung aus Dipropylenglykol und mindestens einem Polyoxypropylen-glykol mit einer Hydroxylzahl bis 400, und maximal 15 Gew.-% Ricinusöl.

10. Transparente, heißdampfsterilisierbare, nicht zytotoxische, im wesentlichen kompakte Polyurethan-Vergußmassen nach Anspruch 1, dadurch gekennzeichnet, daß man als modifizierte Diphenylmethan-diisocyanate (a) Urethangruppen gebunden enthaltende NCO-Prepolymere mit einem NCO-Gehalt von 24 bis 15 Gew.-% verwendet, die hergestellt werden durch Umsetzung von, bezogen auf das Gesamtgewicht, mindestens 85 Gew.-% eines NCO-gruppenhaltigen Prepolymeren mit einem NCO-Gehalt von 29 bis 21 Gew.-%, das seinerseits erhalten wird durch Umsetzung von 4,4'-Diphenylmethan-diisocyanat mit Dipropylen-glykol oder mindestens einem Polyoxypropylen-glykol mit einer Hydroxylzahl von 50 bis 250 oder einer Mischung aus Dipropylenglykol und mindestens einem Polyoxypropylen-glykol mit einer Hydroxylzahl von 50 bis 250, und maximal 15 Gew.-% eines mit Glycerin, Trimethylolpropan oder einer Mischung aus Glycerin und Trimethylolpropan gestarteten Polyoxyalkylen-polyols mit einer Hydroxylzahl von 90 bis 200 und einem Alkaliionengehalt von kleiner als 10 ppm aus der Gruppe der Polyoxypropylen-, Polyoxypropylen-polyoxyethylen- und Polyoxyethylen-polyoxypropylen-polyole.

11. Transparente, heißdampfsterilisierbare, nicht zytotoxische, im wesentlichen kompakte Polyurethan-Vergußmassen nach Anspruch 1, dadurch gekennzeichnet, daß die Aufbaukomponenten (a) und (b) einen Gesamtgehalt als Alkaliionen von 8 bis 110 ppm besitzen.

12. Transparente, heißdampfsterilisierbare, nicht zytotoxische, im wesentlichen kompakte Polyurethan-Vergußmassen nach Anspruch 1, dadurch gekennzeichnet, daß der Katalysator (c) besteht aus Mono-n-octylzinn-(2-ethylhexylthioglykolat), Di-n-octylzinn-bis-(2-ethylhexylthioglykolat) oder Dibutylzinn-dilaurat.

13. Transparente, heißdampfsterilisierbare, nicht zytotoxische, im wesentlichen kompakte Polyurethan-Vergußmassen nach Anspruch 1, dadurch gekennzeichnet, daß man als Katalysator (c) eine Katalisatorkombination verwendet, die besteht, bezogen auf das Gesamtgewicht, aus 1 bis 99 Gew.-% Mono-n-octylzinn-(2-ethylhexylthioglykolat und 99 bis 1 Gew.-% Di-n-octylzinn-bis(2-ethylhexyl-thioglykolat).

14. Transparente, heißdampfsterilisierbare, nicht zytotoxische, im wesentlichen kompakte Polyurethan-Vergußmassen nach Anspruch 1, dadurch gekennzeichnet, daß man als Katalysator (c) eine Katalysatorkombination verwendet, die besteht, bezogen auf das Gesamtgewicht, aus 94 Gew.-% Di-n-octylzinn-bis-(2-ethylhexyl-thioglykolat) und 6 Gew.-% Mono-n-octylzinn-tris-(2-ethylhexylthioglykolat).

15. Verfahren zur Herstellung von transparenten, heißdampfsterilisierbaren, nicht zytotoxischen, im wesentlichen kompakten Polyurethan-Vergußmassen durch Umsetzung von
a) modifizierten Diphenylmethan-diisocyanaten mit einer Viskosität bei 23°C von 100 bis 800 mPas mit
b) Verbindungen mit mindestens zwei reaktiven Wasserstoffatomen
in Gegenwart oder Abwesenheit von
c) Katalysatoren
dadurch gekennzeichnet, daß man als Komponente (b) eine Mischung verwendet, die enthält
b1) 1 bis 20 Gew.-% mindestens eines Polyether-polyols mit einer mittleren Funktionalität von 3 bis 8, einer Hydroxylzahl von 200 bis 1000 und einem Gehalt an Alkaliionen von 150 bis 1200 ppm und
b2) 99 bis 80 Gew.-% Ricinusöl und/oder mindestens eines Polyether-polyols mit einer Funktionalität von 2 bis 3, einer Hydroxylzahl von 90 bis 200 und einem Alkaliionengehalt von kleiner als 10 ppm.

16. Verfahren nach Anspruch 15, dadurch gekennzeichnet, daß die Aufbaukomponenten (a) und (b) in solchen Mengen verwendet werden, daß die Isocyanatkennzahl einen Wert im Bereich von 100 bis 130 besitzt.

17. Verwendung der transparenten, heißdampfsterilisierbaren, nicht zytotoxischen, im wesentlichen kompakten Polyurethan-Vergußmassen nach Anspruch 1 zum Einbetten von Hohlfasern aus Polysulfonen oder Polycarbonaten in Dialysegeräten, zur Herstellung von medizinisch-technischen Artikeln sowie zur Bindung von Biokeramikbeschichtungen auf Endoprothesen.

## Claims

1. A transparent, autoclavable, non-cytotoxic, essentially compact polyurethane embedding composition, prepared by reacting
a) modified diphenylmethane diisocyanates with a viscosity at 23°C of from 100 to 8000 mPas, with
b) compounds with at least two reactive hydrogens in the presence or absence of
c) catalysts
wherein component (b) is a mixture which contains, based on the total weight of (b1) and (b2),
b1) from 1 to 20% by weight of at least one polyether-polyol with an average functionality of from 3 to 8, a hydroxyl number of from 200 to 1000 and an alkali metal ion content of from 150 to 1200 ppm and
b2) from 99 to 80% by weight of castor oil and/or at least one polyether-polyol with a functionality of from 2 to 3, a hydroxyl number of from 90 to 200 and an alkali metal ion content of less than 10 ppm.

2. A transparent, autoclavable, non-cytotoxic, essentially compact polyurethane embedding composition as claimed in claim 1, wherein component (b2) in the mixture is castor oil.

3. A transparent, autoclavable, non-cytotoxic, essentially compact polyurethane embedding composition as claimed in claim 1, wherein component (b) in the mixture is a trifunctional polyether-polyol with a hydroxyl number of from 120 to 180 and an alkali metal ion content of less than 5 ppm.

4. A transparent, autoclavable, non-cytotoxic, essentially compact polyurethane embedding composition as claimed in claim 1, wherein the alkali metal ions in (b1) are potassium ions.

5. A transparent, autoclavable, non-cytotoxic, essentially compact polyurethane embedding composition as claimed in claim 1, wherein the polyether-polyol (b1) is a trimethylolpropane-started polyoxyethylene-polyol with a hydroxyl number in the range from 632 to 970 and a potassium ion content in the range from 400 to 600 ppm.

6. A transparent, autoclavable, non-cytotoxic, essentially compact polyurethane embedding composition as claimed in claim 1, wherein the polyether-polyol (bl) is a glycerol- and/or trimethylolpropane-started polyoxypropylene-polyol with a hydroxyl number in the range from 210 to 480 and a potassium ion content in the range from 400 to 600 ppm.

7. A transparent, autoclavable, non-cytotoxic, essentially compact polyurethane embedding composition as claimed in claim 1, wherein the polyether-polyol (bl) is composed of at least one polyoxypropylene-polyol with an average functionality of from 4 to 8 and a hydroxyl number of from 230 to 500, obtained using sucrose or sorbitol or mixtures of sucrose and sorbitol as starters, possibly also using as costarters water, propylene glycol, glycerol or mixtures of at least two of said costarters, with the proviso that the polyether-polyols have an alkali metal ion content of from 400 to-700 ppm.

8. A transparent, autoclavable, non-cytotoxic, essentially compact polyurethane embedding composition as claimed in claim 1, wherein the polyether-polyol (bl) is a polyoxypropylene- or polyoxyethylene-polyol or a mixture of these polyoxyalkylene-polyols with a hydroxyl number of from 450 to 750, obtained using pentaerythritol or a mixture of pentaerythritol and glycerol and/or trimethylolpropane as starters, with the proviso that the polyether-polyols have an alkali metal ion content of from 150 to 800 ppm.

9. A transparent, autoclavable, non-cytotoxic, essentially compact polyurethane embedding composition as claimed in claim 1, wherein the modified diphenylmethane diisocyanates (a) are NCO prepolymers which contain bonded urethane groups and have an NCO content of from 24 to 15% by weight and are prepared by reacting, based on the total weight, at least 85% by weight of an NCO-containing prepolymer with an NCO content of from 29 to 21% by weight, which in turn is obtained by reacting 4,4'-diphenylmethane diisocyanate with dipropylene glycol or at least one polyoxypropylene glycol with a hydroxyl number of up to 400 or a mixture of dipropylene glycol and at least one polyoxypropylene glycol with a hydroxyl number of up to 400, and a maximum of 15% by weight of castor oil.

10. A transparent, autoclavable, non-cytotoxic, essentially compact polyurethane embedding composition as claimed in claim 1, wherein the modified diphenylmethane diisocyanates (a) are NCO prepolymers which contain bonded urethane groups and have an NCO content of from 24 to 15% by weight and are prepared by reacting, based on the total weight, at least 85% by weight of an NCO-containing prepolymer with an NCO content of from 29 to 21% by weight, which in turn is obtained by reacting 4,4'-diphenylmethane diisocyanate with dipropylene glycol or at least one polyoxypropylene glycol with a hydroxyl number of from 50 to 250 or a mixture of dipropylene glycol and at least one polyoxypropylene glycol with a hydroxyl number of from 50 to 250, and a maximum of 15% by weight of a polyoxyalkylene-polyol which is started with glycerol, trimethylolpropane or a mixture of glycerol and trimethylolpropane and has a hydroxyl number of from 90 to 200 and an alkali metal ion content of less than 10 ppm from the group of polyoxypropylene-, polyoxypropylene-polyoxyethylene and polyoxyethylene-polyoxypropylene-polyols.

11. A transparent, autoclavable, non-cytotoxic, essentially compact polyurethane embedding composition as claimed in claim 1, wherein components (a) and (b) have a total alkali metal ion content of from 8 to 110 ppm.

12. A transparent, autoclavable, non-cytotoxic, essentially compact polyurethane embedding composition as claimed in claim 1, wherein the catalyst (c) is mono-n-octyltin tris(2-ethylhexyl thioglycolate), di-n-octyltin bis(2-ethylhexyl thioglycolate) or dibutyltin dilaurate.

13. A transparent, autoclavable, non-cytotoxic, essentially compact polyurethane embedding composition as claimed in claim 1, wherein the catalyst (c) is a combination composed, based on the total weight, of from 1 to 99% by weight of mono-n-octyltin tris(2-ethylhexyl thioglycolate) and from 99 to 1% by weight of di-n-octyltin bis(2-ethylhexyl thioglycolate).

14. A transparent, autoclavable, non-cytotoxic, essentially compact polyurethane embedding composition as claimed in claim 1, wherein the catalyst (c) is a combination composed, based on the total weight, of 94% by weight of di-n-octyltin bis(2-ethylhexyl thioglycolate) and 6% by weight of mono-n-octyltin tris(2-ethylhexyl thioglycolate).

15. A process for preparing transparent, autoclavable, non-cytotoxic, essentially compact polyurethane embedding compositions by reacting
a) modified diphenylmethane diisocyanates with a viscosity at 23°C of from 100 to 8000 mPas, with
b) compounds with at least two reactive hydrogens in the presence or absence of
c) catalysts
wherein component (b) is a mixture which contains,
b1) from 1 to 20% by weight of at least one polyether-polyol with an average functionality of from 3 to 8, a hydroxyl number of from 200 to 1000 and an alkali metal ion content of from 150 to 1200 ppm and
b2) from 99 to 80% by weight of castor oil and/or at least one polyether-polyol with a functionality of from 2 to 3, a hydroxyl number of from 90 to 200 and an alkali metal ion content of less than 10 ppm.

16. A process as claimed in claim 15, wherein the amounts of components (a) and (b) are such that the isocyanate index is in the range from 100 to 130.

17. Use of the transparent, autoclavable, non-cytotoxic, essentially compact polyurethane embedding compositions as claimed in claim 1 for embedding hollow polysulfone or polycarbonate fibers in dialysis equipment, for producing articles used in medicine, and for bonding bioceramic coatings to endoprostheses.

## Revendications

1. Masses de remplissage ou de scellement en polyuréthanne transparentes, stérilisables à la vapeur chaude, non cytotoxiques, essentiellement compactes, préparées par la réaction
a) de diisocyanates de diphénylméthane modifiés, d'une viscosité à 23°C de 100 à 8000 mPas avec
b) des composés comprenant au moins deux atomes d'hydrogène réactifs,
en présence ou en l'absence
c) de catalyseurs,
caractérisées en ce que l'on utilise, à titre de composant (b), un mélange, qui contient, par rapport au poids total de (b1) et de (b2),
b1) 1 à 20% en poids d'au moins un polyétherpolyol d'une fonctionnalité moyenne de 3 à 8, d'un indice d'hydroxyle de 200 à 1000 et d'une teneur en ions de métaux alcalins de 150 à 1200 ppm, et
b2) 99 à 80% en poids d'huile de ricin et/ou d'au moins un polyétherpolyol d'une fonctionnalité de 2 à 3, d'un indice d'hydroxyle de 90 à 200 et d'une teneur en ions de métaux alcalins inférieure à 10 ppm.

2. Masses de remplissage ou de scellement en polyuréthanne transparentes, stérilisables à la vapeur chaude, non cytotoxiques, essentiellement compactes suivant la revendication 1, caractérisées en ce que le composant de mélange (b2) se compose d'huile de ricin.

3. Masses de remplissage ou de scellement en polyuréthanne transparentes, stérilisables à la vapeur chaude, non cytotoxiques, essentiellement compactes suivant la revendication 1, caractérisées en ce que le composant de mélange (b) est constitué d'un polyétherpolyol trifonctionnel d'un indice d'hydroxyle de 120 à 180 et d'une teneur en ions de métaux alcalins inférieure à 5 ppm.

4. Masses de remplissage ou de scellement en polyuréthanne transparentes, stérilisables à la vapeur chaude, non cytotoxiques, essentiellement compactes suivant la revendication 1, caractérisées en ce que les ions de métaux alcalins dans (b1) se composent d'ions potassium.

5. Masses de remplissage ou de scellement en polyuréthanne transparentes, stérilisables à la vapeur chaude, non cytotoxiques, essentiellement compactes suivant la revendication 1, caractérisées en ce que le polyétherpolyol (b) est un polyoxyéthylènepolyol amorcé au triméthylolpropane, d'un indice d'hydroxyle se situant dans la plage de 632 à 970 et d'une teneur en ions potassium qui varie de 400 à 600 ppm.

6. Masses de remplissage ou de scellement en polyuréthanne transparentes, stérilisables à la vapeur chaude, non cytotoxiques, essentiellement compactes suivant la revendication 1, caractérisées en ce que le polyétherpolyol (b1) est un polyoxypropylènepolyol amorcé à la glycérine et/ou au triméthylolpropane, d'un indice d'hydroxyle qui varie dans la plage de 210 à 480 et d'une teneur en ions potassium qui fluctue dans la plage de 400 à 600 ppm.

7. Masses de remplissage ou de scellement en polyuréthanne transparentes, stérilisables à la vapeur chaude, non cytotoxiques, essentiellement compactes suivant la revendication 1, caractérisées en ce que le polyétherpolyol (b1) se compose d'au moins un polyoxypropylènepolyol d'une fonctionnalité moyenne de 4 à 8 et d'un indice d'hydroxyle de 230 à 500, obtenu en recourant à l'emploi de saccharose ou de sorbite, ou de mélanges de saccharose et de sorbite, à titre de molécules amorceuses, où à titre de coamorceur, on utilise complémentairement et conjointement de l'eau, du propylèneglycol, de la glycérine ou des mélanges d'au moins deux des coamorceurs susmentionnés, avec la condition que les polyétherpolyols possèdent une teneur en ions de métaux alcalins qui fluctue de 400 à 700 ppm.

8. Masses de remplissage ou de scellement en polyuréthanne transparentes, stérilisables à la vapeur chaude, non cytotoxiques, essentiellement compactes suivant la revendication 1, caractérisées en ce que le polyétherpolyol (b1) est un polyoxypropylène- ou polyoxyéthylènepolyol ou un mélange de ces polyoxyalkylènepolyols, d'un indice d'hydroxyle de 450 à 750, que l'on obtient par l'utilisation de la pentaérythrite, ou d'un mélange de pentaérythrite et de glycérine et/ou de triméthylolpropane à titre de molécules amorceuses, avec la condition que les polyétherpolyols possèdent une teneur en ions de métaux alcalins qui varie de 150 à 800 ppm.

9. Masses de remplissage ou de scellement en polyuréthanne transparentes, stérilisables à la vapeur chaude, non cytotoxiques, essentiellement compactes suivant la revendication 1, caractérisées en ce que, à titre de diisocyanates de diphénylméthane modifiés (a), on utilise des prépolymères de NCO contenant en liaison des radicaux uréthanne, d'une teneur en NCO de 24 à 15% en poids, que l'on prépare par la réaction de, par rapport au poids total, au moins 85% en poids d'un prépolymère contenant des radicaux NCO, d'une teneur en NCO de 29 à 21% en poids, que l'on obtient, d'une part, par la réaction du diisocyanate de 4,4'-diphénylméthane avec le dipropylèneglycol ou au moins un polyoxypropylèneglycol, d'un indice d'hydroxyle allant jusqu'à 400, ou d'un mélange de dipropylèneglycol et d'au moins un polyoxypropylèneglycol, d'un indice d'hydroxyle allant jusqu'à 400 et d'au maximum 15% en poids d'huile de ricin.

10. Masses de remplissage ou de scellement en polyuréthanne transparentes, stérilisables à la vapeur chaude, non cytotoxiques, essentiellement compactes suivant la revendication 1, caractérisées en ce que, à titre de diisocyanates de diphénylméthane modifiés (a), on utilise des prépolymères de NCO contenant en liaison des radicaux uréthanne, d'une teneur en NCO de 24 à 15% en poids, qui sont préparés par la réaction de, par rapport au poids total, au moins 85% en poids d'un prépolymère contenant des radicaux NCO, d'une teneur en NCO de 29 à 21% en poids, que l'on obtient, d'une part, par la réaction du diisocyanate de 4,4'-diphénylméthane avec le dipropylèneglycol ou au moins un polyoxypropylèneglycol, d'un indice d'hydroxyle de 50 à 250, ou d'un mélange de dipropylèneglycol et d'au moins un polyoxypropylèneglycol, d'un indice d'hydroxyle de 50 à 250, et d'au maximum 15% en poids d'un polyoxyalkylèneglycol amorcé à la glycérine, au triméthylolpropane, ou à l'aide d'un mélange de glycérine et de triméthylolpropane, d'un indice d'hydroxyle de 90 à 200 et d'une teneur en ions de métaux alcalins inférieure à 10 ppm, appartenant au groupe des polyoxypropylène-, polyoxypropylène-polyoxyéthylène- et polyoxyéthylène-polyoxypropylène-polyols.

11. Masses de remplissage ou de scellement en polyuréthanne transparentes, stérilisables à la vapeur chaude, non cytotoxiques, essentiellement compactes suivant la revendication 1, caractérisées en ce que les composants constitutifs (a) et (b) possèdent une teneur totale en ions de métaux alcalins de 8 à 110 ppm.

12. Masses de remplissage ou de scellement en polyuréthanne transparentes, stérilisables à la vapeur chaude, non cytotoxiques, essentiellement compactes suivant la revendication 1, caractérisées en ce que le catalyseur (c) se compose de (2-éthylhexylthioglycolate) de mono-n-octylétain, de bis-(2-éthylhexylthioglycolate) de di-n-octylétain ou de dilaurate de dibutylétain.

13. Masses de remplissage ou de scellement en polyuréthanne transparentes, stérilisables à la vapeur chaude, non cytotoxiques, essentiellement compactes suivant la revendication 1, caractérisées en ce que l'on utilise, à titre de catalyseur (c), une combinaison catalytique qui se compose, par rapport au poids total, de 1 à 99% en poids de (2-éthylhexylthioglycolate) de mono-n-octylétain et de 99 à 1% en poids de bis-(2-éthylhexylthioglycolate) de di-n-octylétain.

14. Masses de remplissage ou de scellement en polyuréthanne transparentes, stérilisables à la vapeur chaude, non cytotoxiques, essentiellement compactes suivant la revendication 1, caractérisées en ce que l'on utilise, à titre de catalyseur (c), une combinaison catalytique qui se compose, par rapport au poids total, de 94% en poids de bis-(2-éthylhexylthioglycolate) de di-n-octylétain et 6% en poids de tris-(2-éthylhexylthioglycolate) de mono-n-octylétain.

15. Procédé de préparation de masses de remplissage ou de scellement en polyuréthanne transparentes, stérilisables à la vapeur chaude, non cytotoxiques, essentiellement compactes par la réaction
a) de diisocyanates de diphénylméthane modifiés, d'une viscosité à 23°C de 100 à 800 mPas, avec
b) des composés comportant au moins deux atomes d'hydrogène réactifs,
en présence ou en l'absence de
c) des catalyseurs,
caractérisé en ce que l'on utilise, à titre de composant (b), un mélange, qui contient
b1) 1 à 20% en poids d'au moins un polyétherpolyol d'une fonctionnalité moyenne de 3 à 8, d'un indice d'hydroxyle de 200 à 1000 et d'une teneur en ions de métaux alcalins de 150 à 1200 ppm, et
b2) 99 à 80% en poids d'huile de ricin et/ou d'au moins un polyétherpolyol d'une fonctionnalité de 2 à 3, d'un indice d'hydroxyle de 90 à 200 et d'une teneur en ions de métaux alcalins inférieure à 10 ppm.

16. Procédé suivant la revendication 15, caractérisé en ce que l'on utilise les composants constitutifs (a) et (b) en proportions telles que l'indice d'isocyanates possède une valeur qui varie de 100 à 130.

17. Utilisation des masses de remplissage ou de scellement en polyuréthanne transparentes, stérilisables à la vapeur chaude, non cytotoxiques, essentiellement compactes suivant la revendication 1 pour l'incorporation de fibres creuses en polysulfones ou en polycarbonates dans les appareils de dialyse, pour la fabrication d'articles médico-techniques, ainsi que pour la liaison de revêtements biocéramiques à des endoprothèses.
